# EUROPEAN PATENT APPLICATION

(11) **EP 1 250 887 A2**
(43) Date of publication of application: **23.10.2002**
(21) Application number: 02251984.7
(22) Date of filing: 20.03.2002
(51) Int. Cl.: A61B 5/22, G01C 22/00

(54) **Wrist-based fitness monitoring devices**

(30) Priority: 16.04.2001 US 834892
(71) Applicant: Acumen Manufacturing Limited, Kwun Tong, Kowloon (CN)
(72) Inventor: Wong, Philip Lim-Kong, Acumen M. Ltd., U.1902-1903, Kwun Tong, Kowloon, Hong Kong (CN)
(74) Representative: Godwin, Edgar James

(57) **Abstract**

A wrist-based fitness monitoring watch which uses a lap sensing device and a step or a stroke sensing device for detecting movement. In response to the output of a micro controller, information is displayed concerning the number of laps completed, the distance completed, and the time. Also included is a wireless heart rate transfer system which is provided through the micro controller in order to provide heart rate indicators during movement of the user. The lap measurement is based on a synchronization between a heading direction from the lap sensing device and the step/stroke frequency in order to provide the lap count, as well as the distance traveled and the time. Each of these items are accomplished on a wrist based monitoring system which does not require periodic intervention by the user. The system is able to provide split times without the requiring of continual pressing of the start/stop button either before or after a running or swimming event.

## Description

The invention relates to a wrist-based fitness monitoring device and, more particularly, to a watch-type device capable of monitoring one or more exercise and/or physiological related functions.

In performing sports and other fitness activities, it is known to make use of a wrist-based monitoring device, such as a stop watch, to keep track of certain performance measurements, such as the elapsed time of the activity. Other wrist-based devices without a watch function can incorporate both a fitness activity function, such as pedometer measurements, together with a physiological function such as a heart rate monitor. For example, commonly assigned U.S. Patent 5,891,042 discloses a fitness monitoring device having a wireless heart rate monitor together with a pedometer. Such a device includes a microprocessor control unit which displays the indicated functions. The unit can be, for example, a wrist-based device or a clip-on type of device.

In certain fitness activities, such as walking/jogging/ running and swimming, there are some key measurements that are of great interest to the participant if his or her performance is to be measured and improved upon. Some of the most basic of these important measurements in fitness activities are the number of repetitions and the elapsed time of the activity. As discussed above, counters and stop watches are versatile devices which find many applications in sport and fitness training and can be used to keep track of the number of repetitions and elapsed time. However, such known counters and stop watches typically require human intervention for their operation, such as the pressing of a start/stop button at a precise moment both before and after an event occurs. An automatic lap counting circuit is also known from commonly assigned U.S. Patents 5,661,398 and 5,844,960, the specifications of which are expressly incorporated by reference herein.

Despite the above-described known fitness monitoring devices, there is still needed a fitness monitoring device which can combine fitness-related activities with watch-type timing functions and/or with physiological functions.

This, and other needs, are met according to the present invention which provides a wrist-based watch-type of fitness monitoring device incorporating a number of functions heretofore not previously integrated into an easy to use and readily accessible wrist-based device.

In one advantageous embodiment, the present invention provides a fitness monitoring watch to provide timing functions in combination with either a running lap and step counter or a swimming lap and stroke counter. Either of the above combinations can further advantageously be combined with a heart rate monitor in order to provide a complete fitness monitoring device which not only monitors the fitness-related activities but also the physiological aspects of the participant. The incorporation of a heart rate monitor into the watch allows more effective training to occur by factoring into account the participant's heart rate level during the activity.

The present invention advantageously makes use of a micro-controller which receives input signals from the sensing/ monitoring circuits and provides output signals to a display and/or other indicator. The microprocessor is advantageously programmed to process the sensed information into useful activity information such as the number of laps performed, the distance traveled, and the heart rate achieved.

Other objects, advantages and novel features of the present invention will become apparent from the following detailed description of the invention when considered in conjunction with the accompanying drawings.

### BRIEF DESCRIPTION OF THE DRAWINGS

Figure 1 is a general block diagram illustrating a first embodiment of a running lap and step counting heart rate watch-type of fitness monitoring device according to the present invention;

Figure 2 is a schematic circuit diagram illustrating the lap sensing circuitry of Figure 1;

Figure 3 is a schematic circuit diagram of a general step or stroke sensing circuit used in accordance with the present invention;

Figure 4 is a general block diagram of another embodiment according to the present invention for a lap and stroke sensing fitness monitoring device in combination with a wireless heart rate monitor;

Figure 5 is a schematic diagram of a wireless heart rate receiver for use with the present invention;

Figure 6 is a flowchart illustrating the running sensor step and lap processing in accordance with the present invention;

Figures 7a and 7b are flowcharts illustrating the swimming sensor stroke and lap processing in accordance with the present invention; and

Figure 7c is a flowchart illustrating the direction processing subroutine of Figure 7a.

### DETAILED DESCRIPTION OF THE DRAWINGS

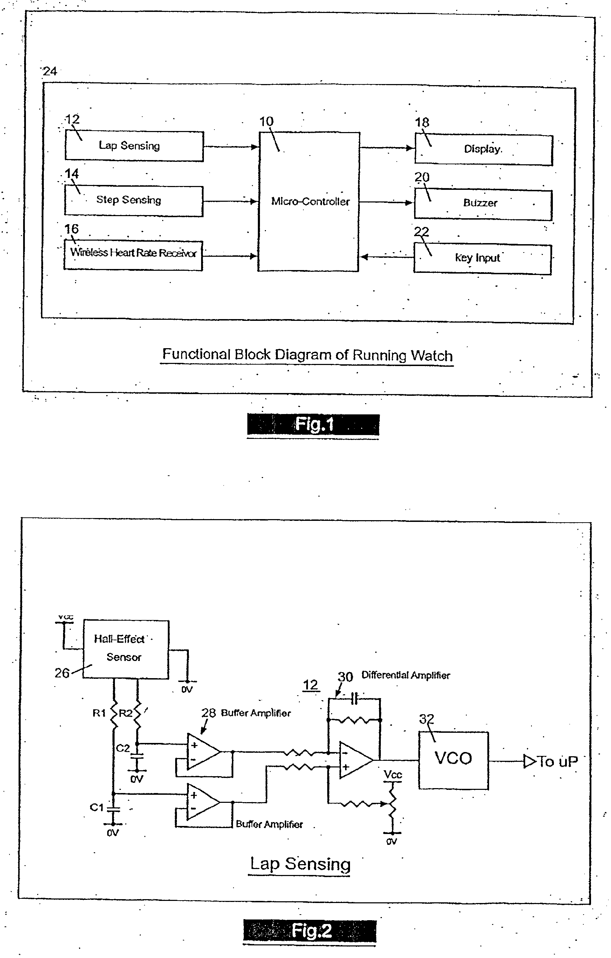

Referring to Figure 1, there is shown a first embodiment of a running lap and step counting heart rate watch 24 according to the present invention. The major components of the wrist-based watch 24 include a micro-controller 10 which receives input signals from a lap sensing circuit 12, a step sensing circuit 14, and a wireless heart rate receiver 16. A key input device 22 also provides input signals to the micro-controller 10. The micro-controller 10 operates in accordance with a program to process the input information in order to provide output signals to at least one of a display 18 or other indicator such as buzzer 20. As a result, the fitness monitoring watch device 24 monitors movements of the user's body via the sport related sensors, i.e., the lap and step sensors 12, 14, while incorporating such monitored movements into a timing device such as a typical watch/stop watch to measure useful performance detail/timing records of the participant during the activity. The lap count and step sensing can be used to automatically log training scores such as the number of laps and split times etc. Advantageously, the invention also includes a heart rate monitor to also detect the physiological aspects of the participant to more effectively perform the activity.

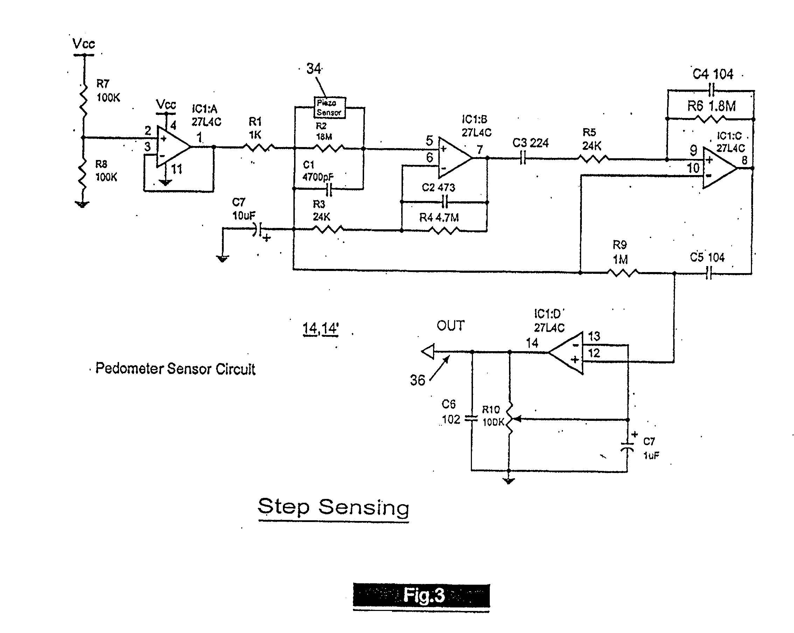

The lap sensing circuit 12 is illustrated in general in Figure 2. This circuit 12 is based on a synchronized voltage controlled oscillating lap counting circuit to resolve the earth's terrestrial magnetism into eight directional bearings such as North, North-East, East, South-East, South, South-West, West and North-West. A Hall-effect sensor element 26 is energized via a supply voltage V_{cc} to pass a current through the element 26. As a result, when a Hall-effect voltage is developed across the Hall-effect sensor in a direction perpendicular to both the direction flow of the energizing current and the direction of the earth's magnetic field, the magnitude and sense of the Hall-effect voltage signal output becomes a function of the relative alignment of the Hall-effect sensor with the magnetic field.

The Hall-effect element 26 provides differential voltage output signals in response to the earth's magnetic field, i.e., the external terrestrial magnetic field strength. The differential voltage output signals are provided through resistors R1, R2 to positive inputs of buffer amplifiers in a buffer amplifier section 28. Capacitors C1, C2 are arranged in series between the respective resistors and a ground potential. The buffer amplifier section 28 operates to hold the differential voltage output signals from the Hall-effect sensor 26.

The differential voltage output signals from the buffer amplifier section 28 are passed through respective resistors and provided as positive and negative inputs to a differential amplifier 30. The differential amplifier 30 converts the input signals into a single output signal with a gain. The single amplified output signal from the differential amplifier 30 is provided as an input to a voltage controlled oscillator 32. The voltage controlled oscillator 32 can be, for example, a model 4046VCO. The voltage controlled oscillator 32 provides an output signal in the form of a digital pulse train having a frequency which varies linearly in accordance with the analog input signal level received at its input. The output signal from the voltage controlled oscillator 32 is provided to the micro-controller 10 (Fig. 1) for processing. A more detailed explanation of lap sensing circuits operable in accordance with the present invention are provided in commonly owned U.S. Patents 5,844,960 and 5,661,398, the specifications of which are expressly incorporated by reference herein.

In summary, the earth's magnetic bearings sensed by the Hall-effect sensor, in terms of voltage level, are voltage-tofrequency converted to increase the resolution. A heading direction is determined/strobed in accordance with a step/stroke frequency to achieve synchronization between the sampled magnetic bearing and coordinated forward movements of the user. A lap count is validated when a cycle of the eight bearings is completed in an orderly manner. Again, reference should be made to U.S. Patents 5,844,960 and 5,661,398 for further details on a lap sensing circuit suitable for the present invention.

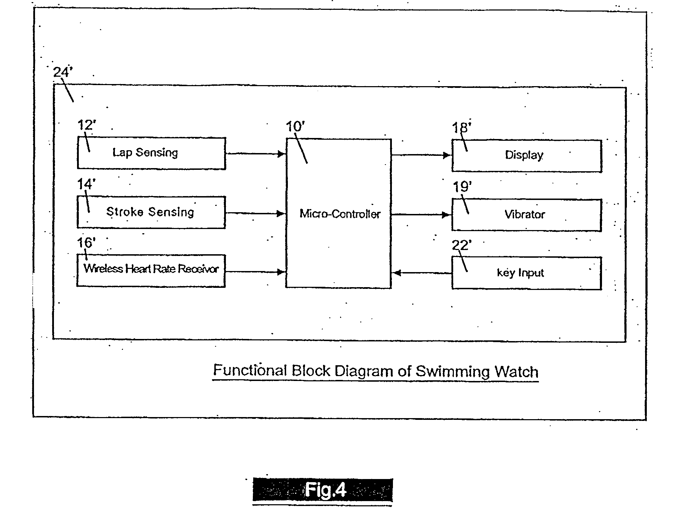

Figure 3 discloses a step/stroke sensing circuit for outputting signals indicative of particular body movements of the user. In the following description, the circuit will be described as a pedometer sensor circuit for detecting a user's steps. Of course, different processing of the output signal from the circuit can be used to detect a user's swimming strokes when used as a swimming stroke sensor. The step sensing circuit 14 is based on a piezo-electric sensor 34 which responds to the user's body motion at each step. The piezo sensor 34 is midbiased via a voltage follower circuit IC1A. The piezo sensor 34 operates in a well-known manner to output a piezo sensor signal 34 in response to detected body motions of the user. The output signal from the piezo sensor 34 is provided to an amplifier circuit IC1B for amplification. The amplified output signal is then further amplified and high frequency limited via the amplifier circuit IC1C. The further amplified signal is fed to a threshold detection circuit IC1D to eliminate non-relevant signals. The result is an output signal 36 which is provided to the micro-controller 10.

The output signal 36 from the pedometer sensor circuit serves two primary functions. First of all, the output signal serves a step counting function to allow the processor to detect the number of steps in order to calculate useful information such as the distance covered. Secondly, the pedometer output signal 36 is used to acquire synchronization with the magnetic bearing resolving circuit (Fig. 2) with each step point direction. This allows the micro-controller 10 to calculate the laps completed.

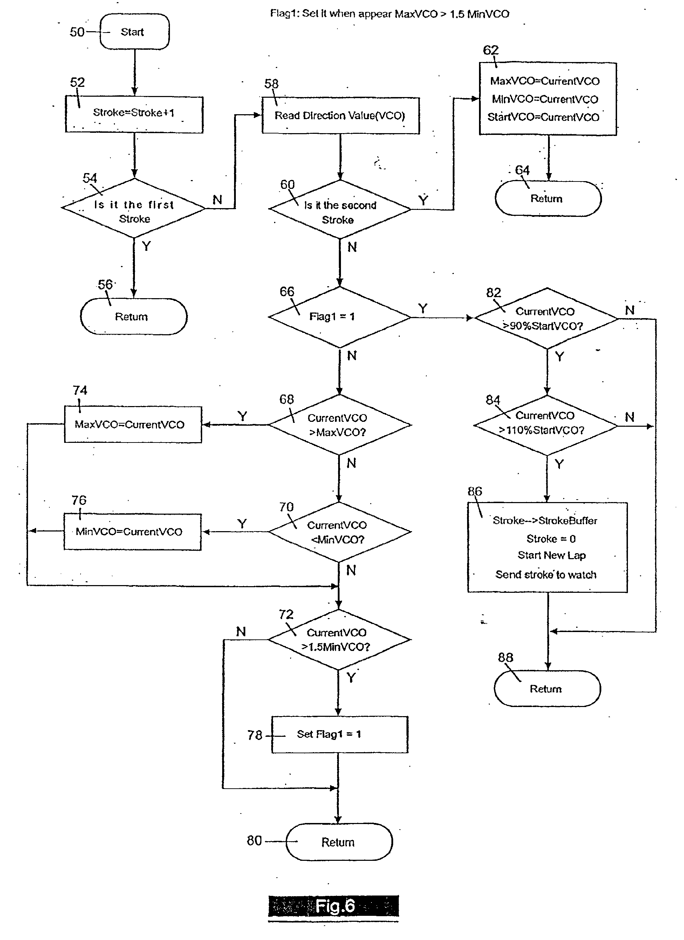

Figure 5 illustrates a wireless heart rate receiver 16 which receives heart-beat signals from, for example, a chest belt transmitter. An example of a wirelessly transmitted heartbeat signal and its reception in a microprocessor of a fitness monitoring device can be found, for example, in commonly owned 2U.S. Patent 5,891,042, the specification of which is expressly incorporated by reference herein.

The wireless heart-rate receiver 16 of Figure 5 is an asic (application specific integrated circuit), which receives a modulated pulse signal from a chest belt transmitter, and provides a decoded digital pulse signal at the asic chip 78 output. This signal is first picked up by a 5 KHz tuned LC tank circuit, buffered and amplified by TR1, and then fed into the input port (pin 6) of the receiver asic. Other external pins to the asic chip are used to set the chip sampling frequency pulse width discrimination for noise filtering. The asic chip uses the latest chip technology in mixed signal processing, filtering, and providing the decoded digital pulse output at pin 13 (DETECT) of the chip. This pulse output is then fed into a micro-controller for heart-rate computation.

The output signal from the wireless heart rate receiver 16 is also fed to the micro-controller 10.

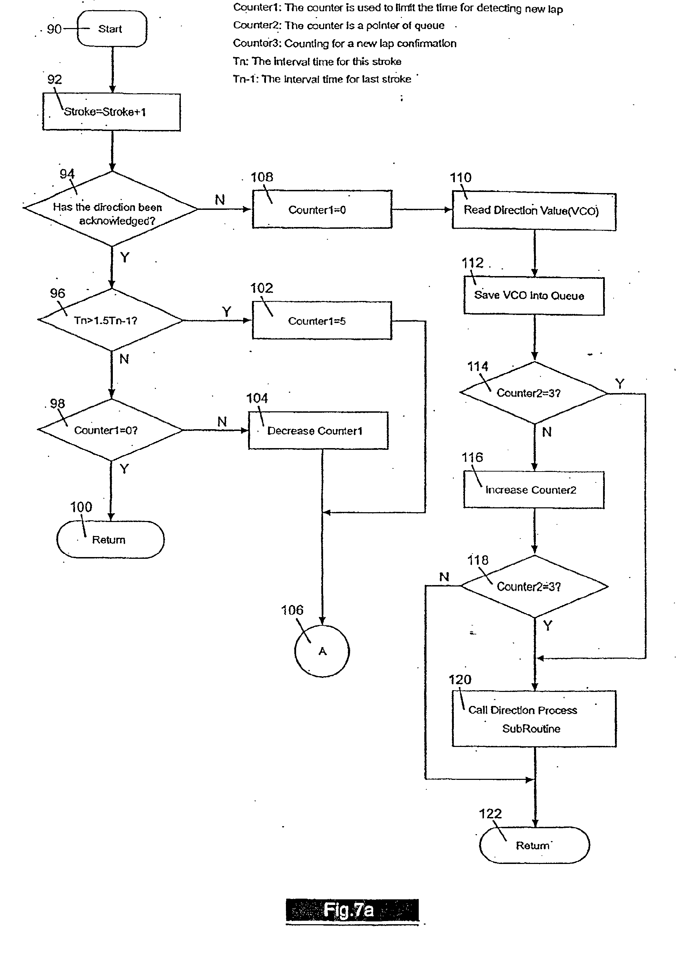

The operation of the micro-controller will now be described with respect to Figure 6. This figure illustrates the operation of the software program for processing the running sensor step and lap signals into useful information for the user.

In Figure 6, the running sensor step/stroke and lap processing operates as follows.

After starting (step 50), the stroke count is incremented (step 52) and then it is determined whether the stroke count indicates that it is the first stroke taken by the user (step 54). If yes, the process returns (step 56) to the start block (step 50). Therefore, after the user initially begins the activity by taking the first stroke, the stroke counter 52 will increment upon the next stroke and then indicate that two strokes have been taken. At block 54, a negative answer will be given, in which case the software reads the direction value from the voltage controlled oscillator (VCO - see Fig. 2) at block 58. Next, the processing inquires as to whether it is the user's second stroke (step 60). In the present situation, this will be answered affirmatively in which the case the processing proceeds to block 62 and sets the max VCO, min VCO and start VCO equal to the current VCO value. The processing then returns as indicated by block 64.

After the above initialization has taken place, the next stroke will result in a negative answer at the inquiry block 60. Therefore, it is determined whether a flag (identified as flag 1) is set (for example to a 1 value) at block 66. If not, then the process determines whether the current VCO value is greater than the max VCO value (step 68). If not, it is next determined whether the current VCO is less than the min VCO (step 70). Again, if not, it is determined whether the max VCO is greater than 1.5 times the min VCO (step 72). If none of the above inquiries (steps 68-72) result in an affirmative answer, then the process returns (step 80) to the start block 50, in which case the flag is not set. This indicates the user has not changed his magnetic heading, such as occurs when running in a straight line. If, however, the current VCO is greater than the max VCO (step 68), then the process sets the max VCO equal to the current VCO (step 74) and then inquires as to whether the max VCO is greater than 1.5 times the min VCO (step 72). Likewise, if the current VCO is less than the min VCO (step 70), the process sets the min VCO equal to the current VCO (step 76) and then determines whether the max VCO is greater than 1.5 min VCO (step 72).

The above processing repeats itself until the user changes his magnetic heading such that the max VCO is greater than 1.5 min VCO (step 72). At that point, the flag is set (step 78). Thus, after the next stroke, the processing will obtain an affirmative answer at step 66, in which case it will then be determined whether the current VCO is greater than 90% of the start VCO value (step 82). If not, the process returns (step 88) to the start block50. If an affirmative answer is obtained, however, it is next determined whether the current VCO is less than 110% of the start VCO (step 84). If not, the process again returns to the start block 50. If affirmative, however, then the process resets the stroke counter to zero and confirms that a new lap is to start (step 86).

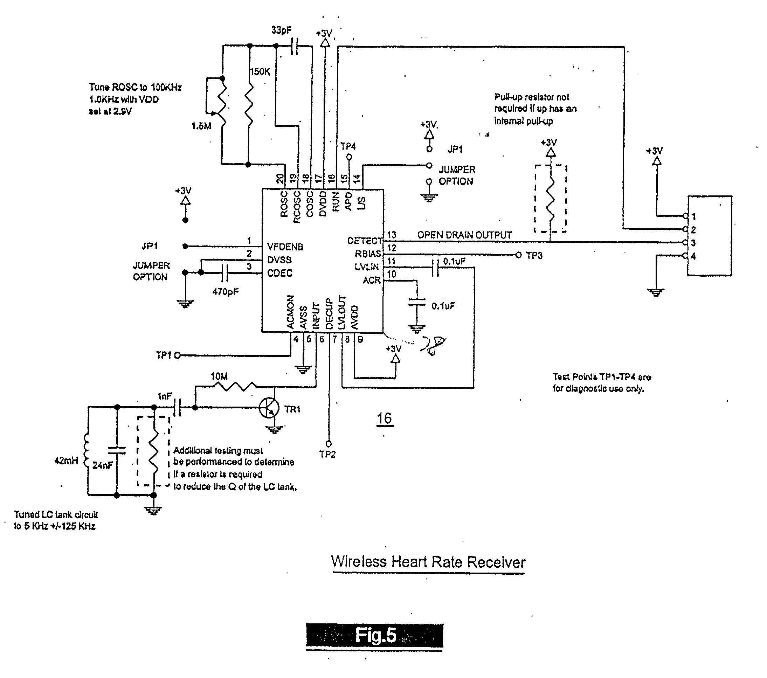

In another embodiment of the present invention shown in Figure 4, in which similar components are given similar reference numbers, the wrist-based device 24' also includes a micro-controller 10', a lap sensing circuit 12', a stroke sensing circuit 14', a wireless heart rate receiver 16', a display 18' and a key input 22'. Because the wrist-based device 24' is used as a swimming lap and stroke counting heart rate watch type of device, the audible indicator described with respect to Figure 1 is replaced by a vibratory indicator 19 coupled to the micro-controller 10'. The vibratory indicator is more likely to obtain the attention of the user when swimming as opposed to the auditory buzzer.

Essentially, the swimming lap and stroke counting heart rate watch operates similarly to the running lap and step counting heart-rate watch with the exception that the lap sensor looks for 180° changes in magnetic bearings (to detect reversal points). Moreover, while the step and stroke sensing circuits both operate using threshold detection, different wave signatures are used to identify strokes versus steps for subsequent decoding. The recognition of the steps versus strokes is performed by the software resident in the micro-controller 10'.

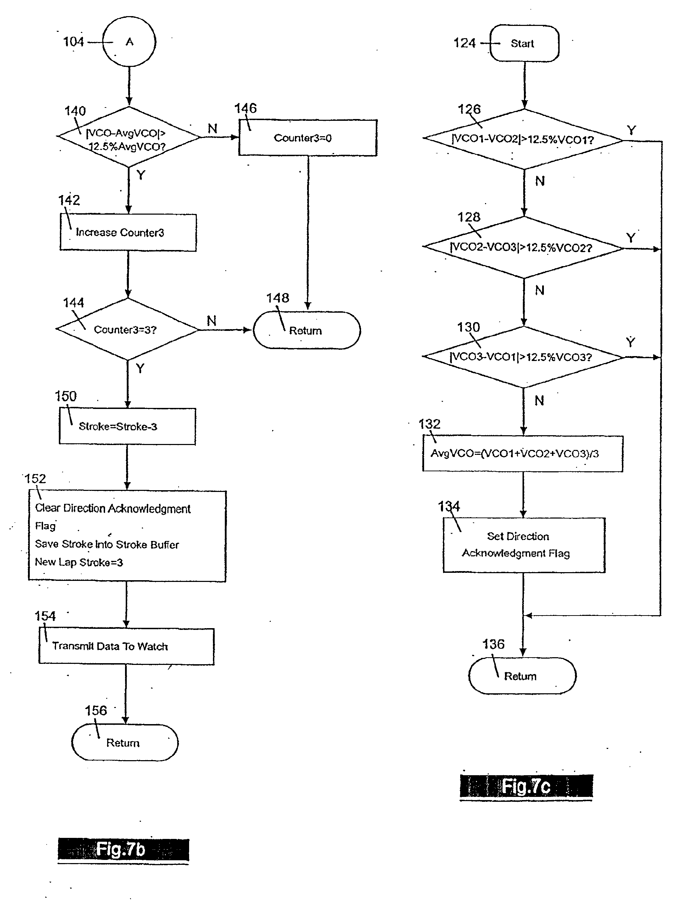

Figures 7a-7c illustrate the swimming sensor stroke and lap processing operation. Referring to Figure 7a, beginning at start block 90, a stroke counter (step 92) is incremented with each stroke. It is then determined whether the direction has been acknowledged (step 94). If yes, then it is determined whether the time interval for the stroke is greater than 1.5 times the time interval of the last stroke (step 96). If not, it is determined whether the first counter equals zero (step 98). If so, the process returns (step 100) to the start block 90. If not, however, then the first counter is decreased (step 104) and the processing continues as will be described with respect to Figure 7b.

Returning to step 94, if the direction has not been acknowledged, then the first counter is set to zero (step 108) and the direction value of the VCO is red (step 110). This value is saved in a cue (step 112). Then, a cue pointing counter is checked to determine whether it is has the value 3 (step 114). If not, the second counter is increased (step 116) and its value is then again checked (step 118). If the second counter's value is still not equal to 3, then the processing returns (step 122) to the start block 90.

After additional passes through the processing steps 108-118, the cue pointing second counter will eventually return an affirmative decision at either step 114 or step 118. In that case, the processing calls the directional process subroutine (step 120). The direction process subroutine 120 will be described below with respect to Figure 7c.

Referring to the direction process subroutine of Figure 7c, the operation starts (block 124) by first determining whether any of a series of conditions (steps 126-130) are met. If not, then an average VCO value is determined (step 132) and the direction acknowledgment flag is then set (step 134). The processing then returns (step 136) to the flowchart of Figure 7a. VCO1, VCO2, and VCO3 are three consecutive VCO readings (stored as variables) to calculate a mean bearing.

Once the operation of Figures 7a and 7c result in the direction acknowledgment flag being set, the inquiry as to whether the direction has been acknowledged (step 94) will provide an affirmative result. In that case, the processing will proceed through steps 96-106 and continue as described below and referenced to Figure 7b.

Figure 7b illustrates the continuation of the processing of Figure 7a. At step 140, it is determined whether the absolute value of the VCO minus the average VCO is greater than 12.5% of the average VCO value. If not, a third counter which counts to confirm a new lap is set to zero (step 146) and the process returns to start block 90. If affirmative, however, the third counter is incremented (step 142) and it is then checked to see whether its value equals 3 (step 144). If not, the process again returns (step 148). However, if an affirmative is obtained at step 144, the stroke value is set equal to the value minus 3 (step 150). Then, the direction acknowledgment flag is cleared, the stroke is saved into the stroke buffer and the new lap stroke is set equal to 3 (step 152). This data is then transmitted to the wrist-based device (step 154) and the process returns (step 156) to the beginning. The final product is a swim watch with a lap/stroke sensors adaptor attached to the watch. The adaptor has a microprocessor on-board to check for lap and stroke data before signaling to the swim watch to update counts. In this way, there are separated batteries for the watch and sensors for easy battery maintenance.

The fitness monitoring device according to the present invention is not limited to the particular embodiments disclosed herein, but rather can include any number of combinations of fitness-related functions and/or physiological measurement functions. For example, a combined watch-pedometer/swim stroke monitor could be provided, in which case some of the circuitry can serve a dual function in accordance with the software control. This combination of fitness-related functions can be included in the watch with or without a heart rate monitor. Of course, other combinations of watch, pedometer, swim stroke monitor, lap counter and heart rate monitor functions can be implemented in accordance with the present invention in a wrist-based device for ease of use and to maximize information available to the user.

The foregoing disclosure has been set forth merely to illustrate the invention and is not intended to be limiting. Since modifications of the disclosed embodiments incorporating the spirit and substance of the invention may occur to persons skilled in the art, the invention should be construed to include everything within the scope of the appended claims and equivalents thereof.

## Claims

1. A user wearable fitness monitoring timing device, comprising:
a lap sensing device outputting a first signal;
a sensor responsive to movement of said user and providing a second signal output;
a wireless heart rate monitor receiving a heart rate signal of said user and providing a third output signal;
a first means for providing a synchronized output signal- in response to said first and second output signal wherein said synchronized output signal indicates a number of laps completed by said user, a distance traveled by said user, a time traveled by said user and relationships between said laps, said distance and said time;
a second means responsive to said third output signal to provide a heart monitor output indicating a heart rate of said user;
a display means for displaying said synchronized output and said heart rate monitor output.

2. The device according to claim 1, wherein said sensor responsive to movement is a step sensor.

3. The device according to claim 1, wherein said sensor responsive to movement of said user is a swimming stroke sensor.

4. The device according to claim 1, wherein said lap sensing device includes a Hall-effect sensor for determining a direction of movement of said user.

5. The device according to claim 1, wherein said sensor responsive to movement includes a piezoelectric sensor.

6. The device according to claim 1, wherein one of said relationships between said laps, said distance and said time is a split time.

7. The device according to claim 4, wherein said lap sensing device includes a voltage controlled oscillator and wherein said first means includes a monitoring means for monitoring the output of said voltage control oscillator to determine the direction of movement of said user.

8. A wrist-based fitness monitoring system worn by a user, said system comprising;
means for monitoring movement of said user as a function of time, distance and direction and outputting a plurality of movement signals;
means for monitoring a physiological condition of said user and outputting a monitoring signal;
control means responsive to said movement signals to synchronize said movement signals to provide a series of relationships between said distance, said time and said direction of movement of said user to include lap completion information and distance completion, wherein said control means also includes means for outputting indicators based on said physiological output; and
display means responsive to said physiological indicators and said relationships output from said controller in order to display said relationships and said physiological indicators.
